# EUROPEAN PATENT APPLICATION

(11) **EP 1 077 071 A1**
(43) Date of publication of application: **21.02.2001**
(21) Application number: 99919582.9
(22) Date of filing: 14.05.1999
(51) Int. Cl.: A61K 51/00, C07D 211/94

(54) **LABELED DRUGS AND NITROXYL COMPOUNDS TO BE USED THEREIN**

(30) Priority: 15.05.1998 JP 13346298
(71) Applicant: DAIICHI RADIOISOTOPE LABORATORIES, LTD., Tokyo 104-0031 (JP)
(72) Inventor: UTSUMI, Hideo, Fukuoka-shi, Fukuoka-ken 810-0032 (JP); SANO, Hiroaki, Chiba 283-0066 (JP); NARUSE, Masaichi, Togane-shi, Chiba-ken 283-0006 (JP); IGARASHI, Takashi, Togane-shi, Chiba-ken 283-0005 (JP); OI, Tetsuo, Sakura-shi, Chiba-ken 285-0817 (JP)
(74) Representative: HOFFMANN - EITLE
(86) International application number: JP9902509
(87) International publication number: WO9959642

(57) **Abstract**

Drugs containing as the active ingredient compounds having a radioactive element and an organic substituent carrying an unpaired electron in a single molecule, for example, labeled nitroxyl compounds represented by general formula (I): 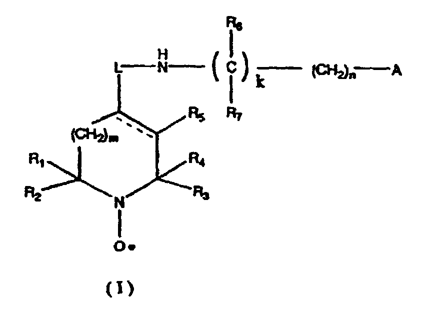 wherein L represents -CO- or -alkylene-CO-, R, R₁, R₂, R₃, and R₄ represent C₁₋₄ alkyl, R₅ represents H, C₁₋₄ alkyl, carboxyl, or lower alkoxycarbonyl, m and k are either 0 or 1, and n is from 1 to 4, R₆ represents methyl or ethyl, R₇ represents H, methyl, ethyl, or carboxyl, A represents an aromatic ring having a radioactive element, and 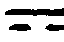 represents either a single or a double bond when m is 0, or a single bond when m is 1.

Use of these drugs makes it possible to easily obtain data about the spin labeling positions, thus providing a means for obtaining exact data relating to active oxygen or free radicals *in vivo* as biological images by nuclear magnetic resonance or biological magnetic measurement.

## Description

### TECHNICAL FIELD

The present invention relates to drugs containing a compound having a radioactive element and an organic substituent carrying an unpaired electron as an active ingredient. Particularly, the present invention relates to drugs containing a radioactive element and an organic substituent carrying an unpaired electron, which is usable in treating ischemic diseases, digestive diseases, cancer, cranial nervous diseases accompanied by nerve degeneration, inflammation, cataract, or drug-induced organopathy caused by *in vivo* active oxygen or free radicals, and is usable in diagnosing diseases relating to *in vivo* active oxygen or free radicals such as tumours and ischemic diseases by acquiring information about active oxygen or free radicals in the brain, heart, or other tissues as a magnetic information.

### BACKGROUND ART

Active oxygen is defined as one type of oxygen species with a short life which is very reactive and takes part in various types of *in vivo* oxidation reactions. The scope of active oxygen varies depending on the definition. In a narrow sense, active oxygen means a hydroxyl radical (·OH), superoxide (O₂·⁻), singlet oxygen (¹O₂), and hydrogen peroxide (H₂O₂). In a broad sense, active oxygen includes a peroxy radical (LOO·) and alkoxy radical (LO·) which are derived from the reaction of the above active species and biological components such as unsaturated fatty acid L, and a hypochlorite ion (ClO⁻) formed from H₂O₂ and Cl⁻ by the reaction with myeloperoxidase and the like.

Radicals are defined as atoms or molecules which possess one or more unpaired electrons. A hydroxyl radical, superoxide, peroxy radical, and alkoxy radical are all radicals. Singlet oxygen and hydrogen peroxide are not radicals, but are formed from a radical reaction or cause other radical reactions.

In recent years, active oxygen and free radicals showing various *in vivo* biological activities have attracted attention and have been studied in the field of biology, medicine, and pharmacology. Active oxygen and free radicals are generated *in vivo* due to ultraviolet rays, radiation, atmospheric pollution, oxygen, metal ions, ischemia-reperfusion, and the like.

Active oxygen and free radicals thus generated cause various *in vivo* reactions such as peroxidization of lipids, denaturation of proteins, and decomposition of nucleic acids. Ischemic diseases, digestive diseases, cancer, cranial nervous diseases accompanied by nerve degeneration, inflammation, cataracts, and drug-induced organopathy are known as diseases accompanied by such phenomena. Noninvasive detection of such active oxygen and free radicals which relate to so many diseases may help in the investigation of the causes of a number of such diseases and provide useful medical information.

The following two methods are known as conventional methods for detecting free radicals. One of these is an indirect method consisting of putting a reagent into a reaction system and detecting the resulting changes in absorbance or emission of light of the reaction system. The other method is an electron spin resonance (ESR) method consisting of directly detecting unpaired electrons of free radicals. Since the ESR method can measure both liquid and solid samples and even opaque or non-uniform samples, this method is very advantageous for detecting active oxygen in collected biological samples or *in vivo*.

However, large amounts of biological samples cannot be measured using conventional ESR devices which utilize microwaves of an X-band (about 9.5 GHz) due to great dielectric loss in water. In recent years, ESR-CT utilizing low-frequency microwaves (300-2000 MHz) has been developed. The development of ESR-CT enables *in vivo* measurement of samples containing a large amount of water, in particular, free radicals in a living body.

Another problem in imaging *in vivo* active oxygen or free radicals is that ESR-CT cannot directly measure active oxygen or free radicals in a living body due to their short life. To solve this problem, a method of indirectly observing *in vivo* active oxygen or free radicals by administering a reagent to a living body and measuring the chemical changes of the reagent caused by active oxygen or free radicals using ESR-CT has been employed. Such a reagent is called a spin trap reagent or a spin label reagent (or spin probe).

The principle of the measurement of active oxygen or the like using a spin trap reagent is shown by the following formula, for example. Specifically, a spin trap reagent with no ESR signal administered to a living body reacts with active oxygen or free radicals and becomes a stable spin adduct with an ESR signal. Therefore, active oxygen or free radicals with a short life can be indirectly observed by measuring the ESR of the spin adduct.

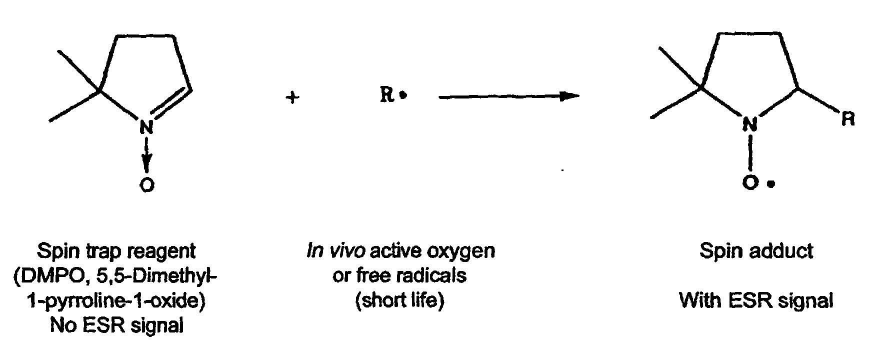

Since the percentage of the spin trap reagent converted into the spin adduct *in vivo* is low, it is difficult to image active oxygen or free radicals by the method using the spin trap reagent due to low sensitivity.

The principle of the measurement of active oxygen or the like using the spin label reagent is shown by the following formula, for example. Specifically, a spin label reagent with an ESR signal administered to a living body reacts with active oxygen or free radicals and becomes stable hydroxylamines with no ESR signal. Active oxygen or free radicals with a short life can be indirectly observed by measuring the attenuation of the ESR.

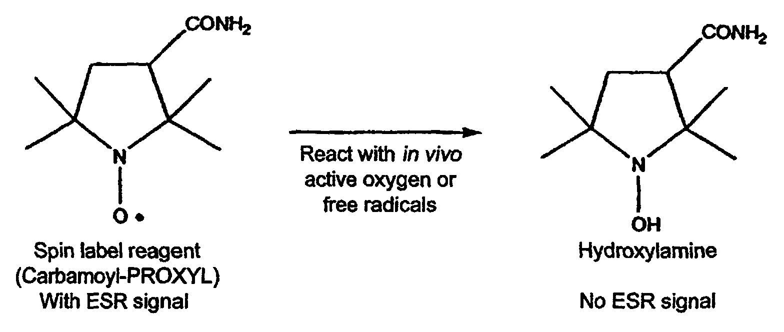

Since the percentage of the spin trap reagent converted into hydroxylamines *in vivo* is high, active oxygen or free radicals can be imaged by the method using the spin label reagent due to high sensitivity.

The principle of the biometry ESR method using the spin label reagent is as follows. Stable radicals administered to a living body are reduced by the reaction with active oxygen or free radicals and lose paramagnetism. *In vivo* active oxygen and free radicals can be noninvasively imaged by measuring and analyzing the signal changes. Therefore, diagnostic reagents containing a stable spin label reagent as ESR contrast media are indispensable for imaging active oxygen or the like using the ESR method.

The principle of a nuclear magnetic resonance (NMR) method was discovered in 1945. In 1973, Lauterbur first applied the NMR method to magnetic resonance imaging (MRI) which is an imaging device for medicine. Since then, the NMR method has progressed remarkably and becomes one of the most universal diagnostic methods at present. MRI first appeared as a diagnostic method using no contrast media. At present, contrast media are used to increase detectability of a lesion site which is difficult to shade. Therefore, contrast media exhibiting superior detectability are demanded.

There are a number of nerve cells in the brain. Changes in the electromotive force accompanied by the activity of the nerve cells are widely used for an electronencephalogram (EEG; brain waves). It is difficult to accurately presume the active site by EEG, because electrical conductivity of a living body greatly varies in the tissue, bone, and the like, and only signals of the potential strength are obtained.

Electric current which is produced by the electromotive force and flows in the nerves forms a magnetic field in the circumference thereof. A record of the magnetic field thus formed is referred to as a magnetoencephalogram (MEG). The strength and direction of the magnetic field are determined depending on the position, strength, and direction of the electric current. Therefore, measurement of the MEG is essentially equivalent to the measurement of the EEG. The MEG measures changes of the magnetic field caused by the biomagnetism of a living body. Since the transmittance of the magnetic field in the biological tissue is constant and almost equal to that in air, the magnetic field is not distorted. Therefore, the active site can be accurately presumed. However, since the amplitude of the magnetic field from the brain is as small as 1/100,000,000 that of the geomagnetism, a magnetism sensor with high sensitivity is required for the measurement. About 20 years ago, a fluxmeter using a superconducting quantum interference device (SQUID) was developed and enabled stable measurement of the biomagnetism of the brain for the first time.

There are a number of nerve cells in the heart similar to the brain. Changes in the electromotive force accompanied by the activity of the nerve cells are widely used for an electrocardiogram (ECG). Electric current, which flows in the nerves caused by the electromotive force, forms a magnetic field in the circumference thereof. A record of the magnetic field thus formed is called a magnetocardiogram (MCG). The magnetic field from the heart of which the amplitude is as small as 1/10,000,000 that of the geomagnetism could be measured for the first time in a stable manner according to the principle of the SQUID using the above fluxmeter.

At present, biomagnetism measuring devices for medicine capable of noninvasively imaging the active site of the brain nerve cells or heart nerve cells from the outside of the body using the fluxmeter have been developed. This biomagnetism measuring method is used to discover the focus of ictus epilepticus and has become a useful examination method for determining whether to apply surgical treatment of epilepsy. This method is expected to be applied to the examination of the initial symptoms of Alzheimer's disease in the future. However, since the magnetic field from the brain or heart is extremely weak, as described above, useful contrast media used in biomagnetic measurement which compensate for the weak magnetic field have been demanded.

In recent years, utility of nitroxyl compounds as contrast media for MRI or ESR and the antioxidation effect thereof have attracted attention. For example, paramagnetic inorganic compounds such as gadolinium are administered as contrast media to contrast the lesion site in the MRI diagnosis in medicine. However, because of the toxicity of such inorganic compounds, nitroxyl compounds have been considered as MRI contrast media which can be used instead of gadolinium. As ESR imaging has been developed and utility thereof has attracted attention, the utility value of nitroxyl compounds as imaging agents has increased. Possibility of utilization of nitroxyl compounds as an active oxygen scavenging agent has also been suggested (see J. Biol. Chem. 263: 17921; 1988).

If information about active oxygen or free radicals in biological tissue can be acquired as biological images by the noninvasive magnetic resonance measuring method, this information is useful for studying pathology in which active oxygen and free radicals take part, such as ischemic diseases, digestive diseases, cancer, cranial nervous diseases accompanied by nerve degeneration, inflammation, cataracts, and drug-induced organopathy (hereinafter referred to as "active oxygen related diseases") and for diagnosing these diseases.

However, imaging using the spin label reagent has the following problems.

When administering a spin label reagent and noninvasively imaging an internal organ twice at certain intervals of time using the ESR-CT, there may be a case where an ESR-CT image obtained in the first imaging disappears in the second imaging. In this case, it is difficult to make a precise diagnosis for two reasons.

Specifically, one is the case where the spin disappears due to active oxygen or free radicals in the internal organ although the spin label reagent is present therein, and the other is the case where the spin label reagent is excreted from the internal organ without being affected by *in vivo* active oxygen or free radicals.

It is necessary to remove part of the internal organ to confirm whether the spin label reagent is present in the internal organ. However, this is impractical in a diagnostic method for a living body.

As described above, it is impossible to precisely determine whether the spin label reagent is present in the objective internal organ using the conventional technique. Therefore, there has been a risk of making a wrong diagnosis.

### DISCLOSURE OF THE INVENTION

The present inventors have conducted extensive studies on a method of acquiring information about the position of the spin label reagent in a living body and about *in vivo* active oxygen and free radicals as biological images using a magnetic resonance method or biomagnetic measuring method. As a result, the present inventors have found that the above problems can be solved by allowing the spin label reagent to carry other labels, specifically, a radioactive element. This finding has led to the completion of the present invention.

The present invention provides a drug composition containing a compound having a radioactive element and an organic substituent carrying an unpaired electron as an active ingredient.

The present invention also provides a method of diagnosing the position of *in vivo* active oxygen or free radicals in a living body of a human or animals other than human or active oxygen related diseases which comprises administering a compound having a radioactive element and an organic substituent carrying an unpaired electron in the molecule to the living body and imaging the compound using gamma rays and ESR, respectively.

Furthermore, the present invention provides a labeled nitroxyl compound shown by the following formula (I) useful for the above drug composition and the diagnosis method: 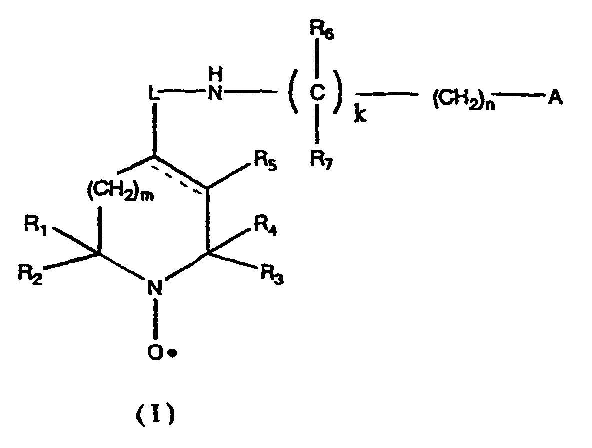 wherein L represents -CO- or -alkylene-CO-, R₁, R₂, R₃, and R₄ individually represent alkyl groups having 1-4 carbon atoms, R₅ represents a hydrogen atom, an alkyl group having 1-4 carbon atoms, carboxyl group, or lower alkoxycarbonyl group, m and k individually represent 0 or 1, n represents an integer from 1 to 4, R₆ represents a methyl group or ethyl group, R₇ represents a hydrogen atom, a methyl group, ethyl group, or carboxyl group, A represents a labeled compound having an aromatic ring, and 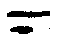 represents either a single bond or a double bond when m is 0, or a single bond when m is 1.

### BEST MODE FOR CARRYING OUT THE INVENTION

The compound having a radioactive element and an organic substituent carrying an unpaired electron in the molecule used in the present invention (hereinafter may be referred to as "labeled spin label compound") is prepared by introducing a radioactive element into a compound having an organic substituent carrying an unpaired electron.

As examples of the labeled spin label compound, a labeled nitroxyl compound shown by the following formula (I) can be given: 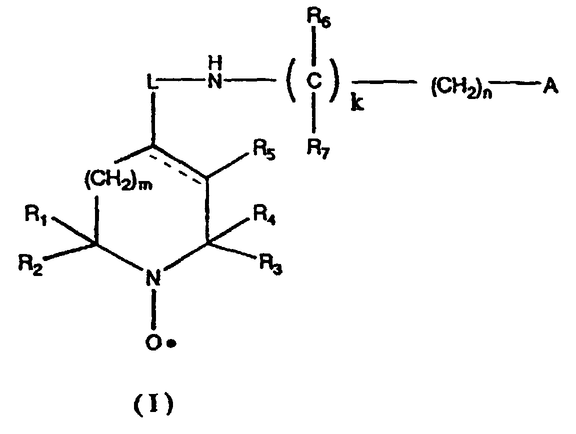 wherein A, L, R₁, R₂, R₃, R₄, R₅, R₆, R₇, m, k, n, and 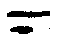 are the same as defined above.

In the labeled nitroxyl compound shown by the above formula (I), as specific examples of the aromatic group A having a radioactive isotopic element, groups shown by the following formulas can be given: 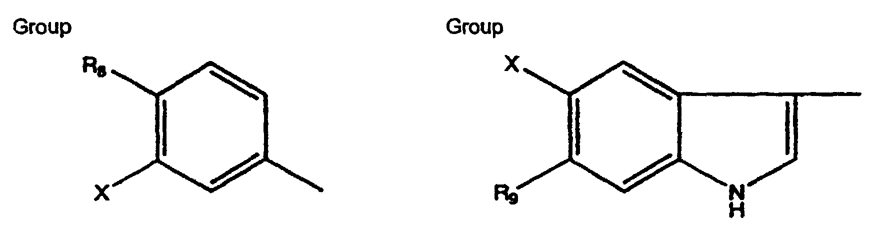 wherein R₈ and R₉ represent a hydrogen atom, a methyl group, ethyl group, hydroxyl group, or carboxyl group, and X represents a radioactive isotope of a halogen atom.

The above compound is obtained by, for example, introducing a radioactive isotopic element into an aromatic group A' having a group which can be replaced by a radioactive isotopic element of a compound shown by the following formula (II) according to the following formula: 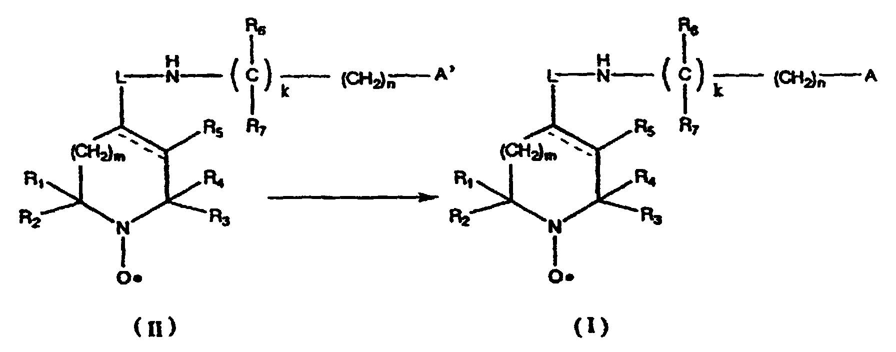 wherein A, L, R₁, R₂, R₃, R₄, R₅, R₆, R₇, m, k, n, and 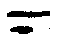 are the same as defined above, and A' represents an aromatic group having a group which can be replaced by a radioactive isotopic element.

As specific examples of the group A' of the compound (II), groups shown by the following formulas can be given: 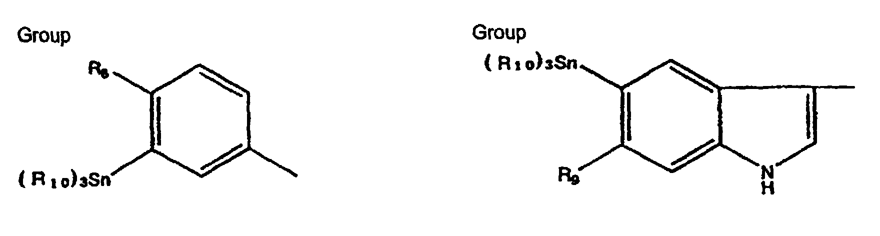 wherein R₈ and R₉ are the same as defined above, and R₁₀ represents an alkyl group having 1-4 carbon atoms; 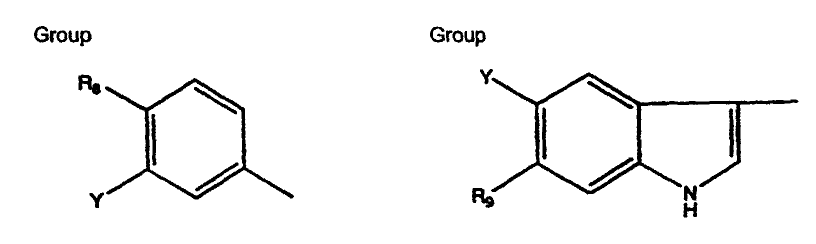 wherein R₈ and R₉ are the same as defined above, and Y represents a stable isotope of a halogen atom.

Among the compound (II), a compound in which the replaceable group in the group A' is a trialkyltin group (compound (IIa)) is prepared by reacting a compound (IV) having a trialkyltin group and an amino group with a carboxyl group of a nitrogen-containing cyclic oxylcarboxylic acid (III) according to the following formula: 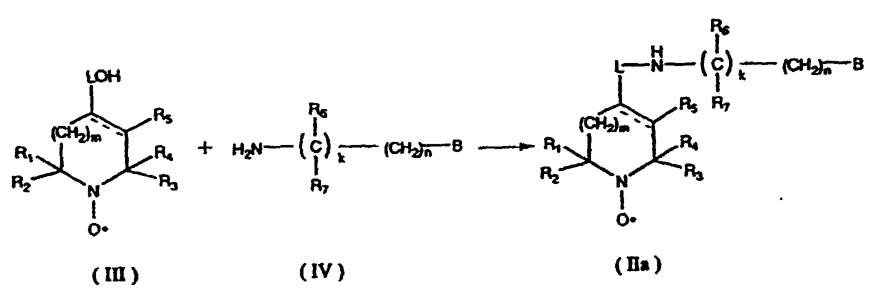 wherein L, R₁, R₂, R₃, R₄, R₅, R₆, R₇, m, n, k, and 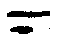 are the same as defined above, and B represents one of the groups shown by the following formulas; 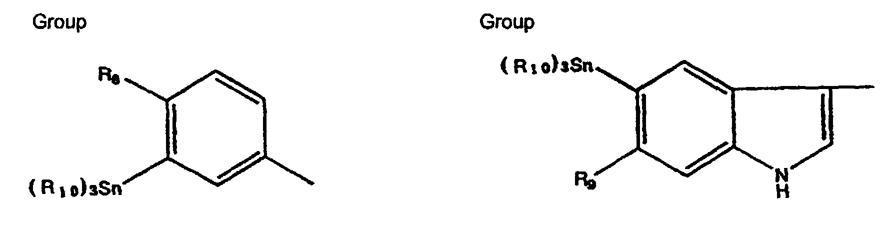 wherein R₈, R₉, and R₁₀ are the same as defined above.

The compound (IV) used as a raw material in the preparation of the compound (IIa) is prepared by reacting a compound in which the group corresponding to the trialkyltin group of the compound (IV) is a bromine (compound (V)) with a bis(trialkyltin) compound such as bisbutyltin in the presence of tetrakis(triphenylphosphine)palladium, according to the following formula: 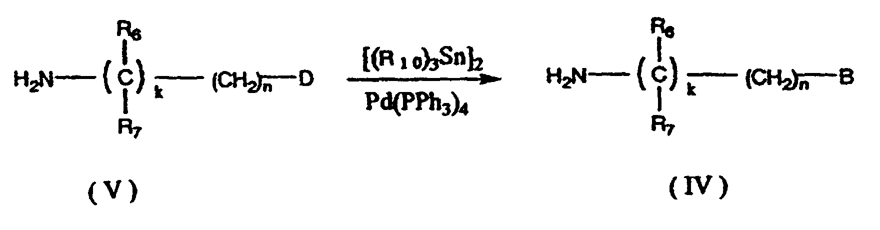 wherein B, R₆, R₇, n, and k are the same as defined above, D represents one of the groups shown by the following formulas, 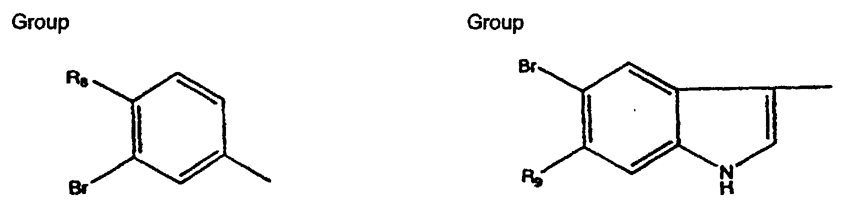 (wherein R₈ and R₉ are the same as defined above), and Ph represents a phenyl group.

The nitrogen-containing cyclic oxylcarboxylic acid (III) used as the other raw material is a compound known in the art. As examples of such a compound, 2,2,5,5-tetramethylpyrrolidinyloxy-3-carboxylic acid, 2,2,6,6-tetramethylpiperidinyloxy-4-carboxylic acid, 2,2,6,6-tetramethylpiperidinyloxy-4-acetic acid, 2-(2,2,6,6-tetramethylpiperidine-N-oxyl-4-yl)proprionic acid, 2-(2,2,5,5-tetramethylpyrrolidinyloxy-3-yl)butyric acid, 2,2,5,5-tetramethylpyrrolidinyloxy-3,4-dicarboxylic acid, and the like can be given.

Among the compound (II), a compound in which the replaceable group in the group A' is a stable isotope of halogen (compound (IIb)) is prepared by reacting a compound (VI) having an amino group with a carboxyl group of the nitrogen-containing cyclic oxylcarboxylic acid (III): 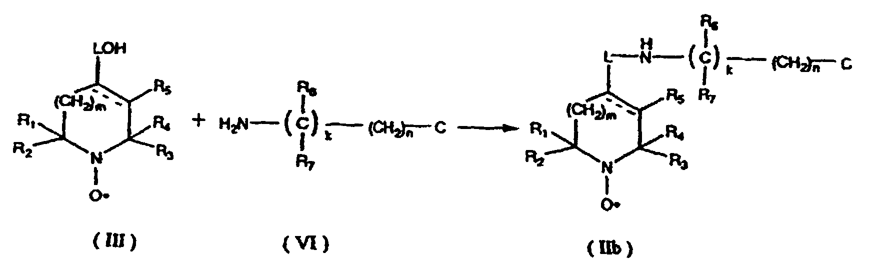 wherein L, R₁, R₂, R₃, R₄, R₅, R₆, R₇, m, n, k, and 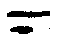 are the same as defined above, and C represents one of the groups shown by the following formulas; 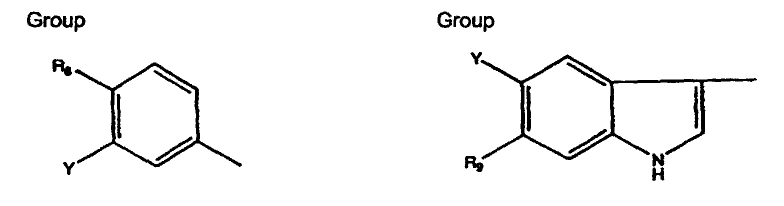 wherein Y, R₈, and R₉ are the same as defined above.

The compound (VI) used in this reaction is also known in the art. Examples include 3-iodoaniline, L-3-iodo-α-methylthyroxine, 5-iodo-DL-tryptophan, and the like.

The compound (I) is obtained from the intermediate (II) thus prepared using a conventional method.

Specifically, the compound (I) is obtained from the compound (IIa) by oxidizing a trialkyltin group of the compound (IIa) using an oxidizing agent such as chloramine T and replacing the trialkyltin group by a radioactive isotope of a halogen atom, according to the following formula: 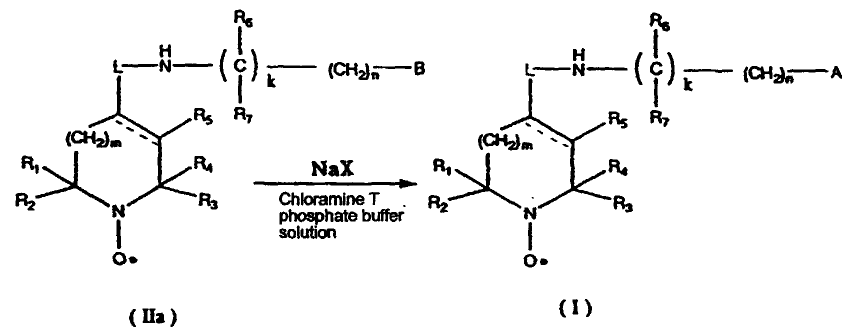 wherein A, B, L, X, R₁, R₂, R₃, R₄, R₅, R₆, R₇, m, n, k, and 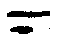 are the same as defined above.

The compound (I) is also obtained by dissolving the compound (IIb) and an alkaline ion salt of a radioactive isotope of a halogen atom (for example, NaX)) in an appropriate solvent, thereby causing an isotope exchange reaction at room temperature or while heating according to the following formula: 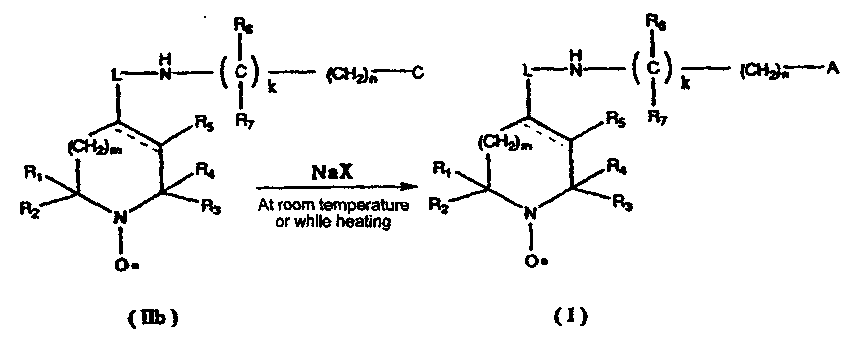 wherein A, C, L, X, R₁, R₂, R₃, R₄, R₅, R₆, R₇, m, n, k, and 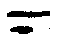 are the same as defined above.

A drug composition is prepared using the compound having a radioactive element and an organic substituent which contains an unpaired electron in the molecule, such as the nitroxyl compound (I), by dissolving the compound in a pharmaceutically or chemically acceptable solvent such as a physiological saline solution or isotonic phosphate buffer solution and adding optional components such as propylene glycol or benzyl alcohol, as required.

The drugs of the present invention are preferably prepared as injections, drops, liniments, eye drops, and the like.

As examples of the usage of the drugs of the present invention, diagnostic drugs can be given. Such diagnostic drugs are used for diagnosing active oxygen related diseases by means of detecting the presence of active oxygen or free radicals by intravascular administration of the drug. For example, the diagnostic drugs are used as contrast media for MRI of brain or heart diseases, biomagnetic measurement, and ESR.

The amount of the nitroxyl compound (I) to be used in the drugs may differ depending on the object or objective internal organs or diseases. The drugs are generally administered so that the amount of the nitroxyl compound (I) is from 1 ng/kg to 500 mg/kg, for example.

As examples of other usage of the drugs, preventives or therapeutic agents for diseases caused by active oxygen or *in vivo* radicals can be given. Such preventives or therapeutic agents, which react with active oxygen or free radicals and eliminate them, are effective for prophylaxis and treatment of the active oxygen related diseases.

The drugs administered to normal experimental animals or disease model experimental animals can externally detect and image active oxygen or free radicals generated from tissues or internal organs in a normal or diseased state. The drugs can be used as detection reagents for determining what active oxygen and free radicals relate to what kind of diseases from the results of imaging, thereby providing useful medical information.

Furthermore, the drugs can be used as detection reagents for measuring the presence or absence or the amount of active oxygen or free radicals in biological tissues by homogenizing collected samples, adding an appropriate buffer solution and the drugs to the homogenized solution, allowing the mixture to react for a certain period of time, and measuring the ESR.

### EXAMPLES

The present invention will be described in more detail by examples, which should not be construed as limiting the present invention.

### Example 1

### Synthesis of 3-(2-(3-tri-n-butylstanylphenyl))-2,2,5,5-tetramethylpyrrol idin-1-yloxy:

### (1) Synthesis of 3-tri-n-butylstanylaniline

3.16 ml (29.1 mmol) of 3-bromoaniline was dissolved in 50 ml of toluene. Argon gas was bubbled through the solution for 5 minutes. After the addition of 17.6 ml (34.9 mmol) of bis(tributyltin) and 336 mg (0.291 mmol) of tetrakis(triphenylphosphine)palladium in that order, the mixture was refluxed for six hours while heating. The reaction mixture was diluted with 100 ml of ethyl acetate. The mixture was then washed with water and dried (MgSO₄) and the solvent was evaporated. The residue was purified by flash column chromatography (silica gel, hexane-ethyl acetate, 4:1) to obtain 7.30 g of 3-tri-n-butylstanylaniline as a reddish orange oily substance (yield: 66%).
IR(cm⁻¹):
   3470, 3360, 2950, 2910, 2870, 2850, 1610, 1580, 1480, 1460, 1430, 1380, 1280, 1260-1210, 1160, 1080, 1000, 880, 870, 780
¹H-NMR:
   0.40-1.16 (27H, m), 3.56 (2H, brs), 6.55-7.25 (4H, m)

### (2) Synthesis of 3-(2-(3-tri-n-butylstanylphenyl))-2,2,5,5-tetramethylpy rrolidin-1-yloxy

1 g (3.57 mmol) of 3-carboxy-PROXYL was dissolved in 8 ml of dichloromethane. After the addition of 1.11 g (5.37 mmol) of dicyclohexylcarbodiimide at 0°C, the mixture was stirred at room temperature for two hours. The reaction mixture was diluted with 20 ml of dichloromethane. The mixture was then washed with 5 ml of 3N-HCl, 5 ml of a saturated sodium hydrogencarbonate aqueous solution, and water. The solution was dried (MgSO₄) and the solvent was evaporated. The residue was purified by flash column chromatography (silica gel, hexane-ethyl acetate, 9:1) to obtain 924 mg of 3-(2-(3-tri-n-butylstanylphenyl))-2,2,5,5-tetramethylpyrrol idin-1-yloxy as a reddish orange oily substance (this compound may be called "DRD278") (yield: 56%).
IR(cm⁻¹):
   3320, 2960, 2930, 2880, 2850, 1700, 1660, 1600, 1580, 1540, 1480, 1460, 1410, 1380, 1370, 1310, 1200, 1190, 1140, 1100, 790
FAB-MASS (m/z; (relative strength))
   551 (M⁺, 34), 479 (13), 423 (13), 365 (14), 294 (15), 266 (8), 235 (30), 212 (28), 179 (100), 152 (16), 110 (48), 90 (35)

### (3) Synthesis of 3-(2-(3-I-129-phenyl))-2,2,5,5-tetramethylpyrrolidin-1-yloxy

1 g (5.37 mmol) of 3-carboxy-PROXYL was dissolved in 8 ml of dichloromethane. After the addition of 1.11 g (5.37 mmol) of dicyclohexylcarbodiimide at 0°C, the mixture was stirred at 0°C for fifteen minutes. After the addition of 640 µl (5.37 mmol) of 3-iodoaniline at 0°C, the mixture was stirred at room temperature for two hours. The reaction mixture was diluted with 20 ml of dichloromethane. The mixture was washed with 5 ml of 3N-HCl, 5 ml of a saturated sodium bicarbonate aqueous solution, and then water. The solution was dried (MgSO₄) and the solvent was evaporated. The residue was purified by flash column chromatography (silica gel, hexane-ethyl acetate, 9:1) to obtain 401 mg of 3-(2-(3-I-129-phenyl))-2,2,5,5-tetramethylpyrrolidin-1-ylox y as a light yellow needle-like crystal (yield: 26%).
IR(cm⁻¹):
   3230, 2980, 2940, 2860, 1710, 1670, 1530, 1470, 1450, 1430, 1400, 1370, 1340, 1320, 1260, 1220, 1200, 1170, 1130, 1100, 1080, 1000, 970, 900, 760
FAB-MASS (m/z; (relative strength))
   389 (29), 225 (42), 188 (100), 154 (24), 136 (19), 111 (11), 98 (16), 74 (34)

### Example 2

### Preparation of 3-(2-(3-I-123-phenyl))-2,2,5,5-tetramethylpyrrolidin-1-ylox y for injection:

1 ml of an ethanol solution of DRD278 (concentration: 1 mg/ml), 500 µl of a chloramine T aqueous solution (concentration: 10 mg/ml), 40 µl of a 0.4M-phosphate buffer solution (pH: 1.3), and 100 µl of a NaI-123 aqueous solution (3.7 gBq/ml) were mixed and stirred at room temperature for 5 minutes. After the addition of 125 µl of a sodium hydrogensulfite aqueous solution (concentration: 100 mg/ml) and 1 ml of a saturated sodium bicarbonate aqueous solution, the mixture was shaken gently. 5 ml of ethyl acetate was then added and the mixture was shaken. An organic layer was fractionated and loaded onto a sep-pak cartridge to collect eluants, 1 ml each. Then, 5 ml of ethyl acetate was loaded onto a sep-pak cartridge to collect eluants, 1 ml each. Radioactivity of ten fractions thus obtained was measured to select the fractions with high radioactivity. The solvent was evaporated from the fractions by blowing nitrogen therethrough at 50°C. 740 µl of ethanol-physiological saline solution-Tween 80 (1:8:1) was added to the residue to dissolve the residue. As a result of a purity test (TLC method, stationary phase: silica gel, mobile phase: hexane-ethyl acetate (1:2), determined by measuring radioactivity), the purity of the solution was 89%. This solution of 3-(2-(3-I-123-phenyl))-2,2,5,5-tetramethylpyrrolidin-1-ylox y was used as an injection.

### Example 3

### Biodistribution test using ESR:

A biodistribution test was carried out by ESR using 3-(N-(3-iodo-127-1-phenyl))-2,2,5,5-tetramethylpyrrolidinoy 1-3-carboxamide as a test sample (test sample A).

The test was carried out as described below using six mice (ddY, female) as experimental animals. 250 µl of the test sample A (concentration was adjusted to 1 mg/ml by dissolving the test sample in a mixed solution of physiological saline solution-ethanol-Tween 80 (8:1:1)) was administered to the abdominal cavities of the mice without using anaesthesia and allowed to stand for 30 minutes. The mice were then quickly dehematized and the liver, kidneys, spleen, and brain were removed. The liver, kidneys, and brain were diluted with a phosphate buffer solution in an amount equivalent to the weights of these removed internal organs and were then homogenized. The spleen was diluted with a phosphate buffer solution in an amount twice the weight of the removed spleen and was then homogenized. The blood was diluted with a phosphate buffer solution in the same amount as that of the blood. These samples were used as non-reoxidized ESR samples.

Part of the tissues thus treated was removed and a potassium ferricyanide solution was added in an amount equivalent to the weights of the removed tissues. The samples which had become hydroxylamines by *in vivo* reduction or the like were reconverted to nitroxide radicals (one electron oxidation) to prepare reoxidized ESR samples.

The concentrations of the reoxidized ESR samples and non-reoxidized ESR samples were measured by X-band ESR. A test sample solution in an amount equal to that administered to the mice was prepared. The solution was treated in the same manner except for adding the nine-fold phosphate buffer solution. The concentration of the test sample A was measured by x-band ESR, which was taken as the total dosage. 15 mm of each sample was put into a 100 µl capillary (length 116.5 mm) and was then placed in a quartz cell to measure the ESR spectrum under the following conditions.

### (Measurement conditions)

Measuring device: electron spin resonance spectrometry "JES-3000" (manufactured by JEOL Ltd.)
Sensitivity: 1 x 10¹¹ Spins/Gauss
Resolution: 500 mGauss
Magnetic field modulation frequency: 100 kHz
Magnetic field sweep width: 100 Gauss
Magnetic field modulation width: 8 Gauss
Integration function: build-in

### (Results)

The ratio of the test sample A in the internal organs and blood to the total dosage (%dose) and the value obtained by dividing the %dose by the weight of the internal organs and blood (%dose/g) were calculated from the ESR spectrum of the reoxidized ESR samples and non-reoxidized ESR samples. The results are shown in Tables 1 and 2 together with the standard deviations thereof.

**TABLE 1**

| Measurement results of non-reoxidized sample (N = 4) | | | | |
|---|---|---|---|---|
| Internal organ | %dose | | %dose/g | |
| | Average | Standard deviation | Average | Standard deviation |
| Blood | 0.16 | 0.13 | 0.08 | 0.07 |
| Liver | 0 | 0 | 0 | 0 |
| Kidneys | 0 | 0 | 0 | 0 |
| Spleen | 0.02 | 0.02 | 0.21 | 0.23 |
| Brain | 0 | 0 | 0 | 0 |

**TABLE 2**

| Measurement results of reoxidized sample (N = 4) | | | | |
|---|---|---|---|---|
| Internal organ | %dose | | %dose/g | |
| | Average | Standard deviation | Average | Standard deviation |
| Blood | 0.92 | 0.24 | 0.43 | 0.12 |
| Liver | 2.86 | 0.54 | 2.30 | 0.42 |
| Kidneys | 0.38 | 0.05 | 1.34 | 0.18 |
| Spleen | 0.07 | 0.02 | 0.72 | 0.05 |
| Brain | 0.18 | 0.05 | 0.41 | 0.09 |

As is clear from Table 2, the biodistribution of the test sample A, including the amount of the samples reduced to hydroxylamines, was as high as 2.86 %dose in the liver. However, unconverted test sample A (not reduced to hydroxylamines and remaining in the same chemical form) was not detected in the liver, as shown in Table 1. Specifically, since the hydroxylamines of the test sample A accumulated in the liver at a ratio of 2.86 %dose had no ESR signal, these hydroxylamines could not be detected by ESR.

### Example 4

### Biodistribution test using RI:

A biodistribution test using a gamma counter was carried out using a mixture (test sample C) of 3-(N-(3-iodo-123-1-phenyl))-2,2,5,5-tetramethylpyrrolidinoy l-3-carboxamide (test sample B) and 3-(N-(3-iodo-127-1-phenyl))-2,2,5,5-tetramethylpyrrolidinoy l-3-carboxamide which was the carrier thereof (test sample A).

The test was carried out as described below using four mice (ddY, female) as experimental animals. 120 mg of the test sample C (dissolved in a mixed solution of physiological saline solution-ethanol-Tween 80 (8:1:1) so that the concentration was 1 mg/ml) was administered to three mice anesthetized with Nembutal (50 mg/kg). The mice were allowed to stand for 30 minutes and then quickly dehematized. The liver, kidneys, spleen, stomach, small intestine, large intestine, bladder, lungs, ovaries, brain, thyroid gland, heart, muscle, and bone were removed. The removed internal organs were allowed to stand in a vial for six days to attenuate the radioactivity levels to obtain gamma counter samples of the internal organs.

Radioactivity of the samples after attenuation was measured using a gamma counter. As a standard, radioactivity of NaI-123 diluted with a physiological saline solution (diluted so that radioactivity was one tenth of dosage) was measured using a Capintec and a gamma counter. Radioactivity of the administered test sample C was measured using a Capintec. Radioactivity was converted into a value by a gamma counter using a coefficient calculated from the measured value of the standard sample, which was taken as a standard for the total dosage.

### (Results)

The ratio of the test sample in the internal organs and blood to the total dosage (%dose), and the value obtained by dividing the %dose by the weight of the internal organs and blood (%dose/g) were calculated from the measurement results. The results are shown in Table 3, together with the standard deviations thereof.

**TABLE 3**

| Results of biodistribution test | | | | |
|---|---|---|---|---|
| Internal organ | %dose | | %dose/g | |
| | Average | Standard deviation | Average | Standard deviation |
| Blood | 5.33 | 0.25 | 2.66 | 0.12 |
| Liver | 14.56 | 1.11 | 12.77 | 0.44 |
| Kidneys | 1.77 | 0.45 | 6.13 | 1.21 |
| Spleen | 0.349 | 0.08 | 2.88 | 0.50 |
| stomach | 2.60 | 0.17 | 5.49 | 0.89 |
| Small intestine | 17.26 | 2.81 | 12.61 | 1.91 |
| Large intestine | 2.164 | 0.46 | 2.60 | 0.41 |
| Bladder | 4.25 | 1.09 | 32.52 | 8.34 |
| Lungs | 0.78 | 0.07 | 4.068 | 0.72 |
| Ovaries | 0.35 | 0.298 | 5.28 | 4.13 |
| Brain | 0.54 | 0.13 | 1.256 | 0.19 |
| Thyroid gland | 0.10 | 0.01 | 7.22 | 0.54 |
| Heart | 0.26 | 0.03 | 2.16 | 0.16 |
| Muscle | 0.24 | 0.11 | 1.47 | 0.17 |
| Bone | 0.05 | 0 | 1.45 | 0.20 |

As is clear from Table 3, the test sample C was distributed in the small intestine at the highest ratio of 17.26 %dose, followed by 14.56 %dose in the liver.

### INDUSTRIAL APPLICABILITY

The compound having a radioactive element and an organic substituent carrying an unpaired electron, which is used as an active ingredient of the drugs of the present invention, is useful for acquiring biological images of the distribution of free radicals by a magnetic resonance method, since the organic substituent carrying an unpaired electron interacts with *in vivo* active oxygen or free radicals and the presence of the compound thereof can be determined by the radioactive element. Therefore, the compound is suitably used for accurately diagnosing the active oxygen related diseases or symptoms such as ischemic diseases, digestive diseases, cancer, cranial nervous diseases accompanied by nerve degeneration, inflammation, cataracts, or drug-induced organopathy in which active oxygen or free radicals take part.

Specifically, the active oxygen related diseases can be diagnosed by administering a composition containing the compound to a living body as a diagnosing reagent, and detecting the signal change of the compound *in vivo* by ESR, NMR, and the like. The nitroxyl compound of the present invention interacts with *in vivo* free radicals and contains an element which emits gamma rays. Therefore, the accurate reaction position of the nitroxyl compound with *in vivo* free radicals can be noninvasively imaged by using a magnetic resonance method and gamma rays.

Therefore, the diagnostic reagent of the present invention is used for MRI and biomagnetic measurement. Moreover, if ESR devices capable of measuring large capacity biological samples such as a human body are developed in the future, diseases or symptoms in which active oxygen or free radicals take part are noninvasively diagnosed using the diagnostic reagent by acquiring the images of *in vivo* free radical distribution by the ESR method.

Since the compound having a radioactive element and an organic substituent carrying an unpaired electron of the present invention such as the nitroxyl compound reacts with *in vivo* active oxygen or free radicals and eliminates them, the compound can be used as preventives or therapeutic agents for active oxygen related diseases such as digestive diseases, ischemic diseases, cancer, cranial nervous diseases accompanied by nerve degeneration, inflammation, cataracts, or drug-induced organopathy in which active oxygen or free radicals take part.

With the compound it is possible to externally detect and image active oxygen or free radicals generated from tissues or internal organs in a normal or diseased state by administering the compound to normal experimental animals and disease model experimental animals. From these results, the compound can be used as detection reagents for detecting which active oxygen and free radicals relate to what kind of diseases, whereby useful medical information is obtained.

Furthermore, the presence or absence or the amount of active oxygen or free radicals in biological tissue can be measured by homogenizing collected samples, adding an appropriate buffer solution and the compound to the samples, reacting the mixture for a certain period of time, and measuring the signal strength by ESR.

## Claims

1. A drug composition comprising a compound which comprises a radioactive element and an organic substituent carrying an unpaired electron in the molecule as an active ingredient.

2. The drug composition according to claim 1, which is used to measure and detect *in vivo* active oxygen or free radicals.

3. The drug composition according to claim 2, which is used as a contrast medium used to image the distribution of *in vivo* active oxygen or free radicals.

4. The drug composition according to any one of claims 1 to 3, which is used as a contrast medium for ESR, NMR, biomagnetic measurement, and nuclear medicine diagnosis.

5. The drug composition according to claim 1, which is used to scavenge *in vivo* active oxygen or free radicals.

6. The drug composition according to claim 1 or 5, which is used as a diagnosing reagent or therapeutic agent for ischemic diseases, digestive diseases, cancer, cranial nervous diseases accompanied by nerve degeneration, inflammation, cataract, and drug-induced organopathy.

7. A method of noninvasively identifying the accurate reaction position of *in vivo* active oxygen or free radicals in a living body of a human or animals other than human, which comprises administering a compound having a radioactive element and an organic substituent carrying an unpaired electron in the molecule to the living body and imaging the compound using gamma rays and ESR, respectively.

8. A labeled nitroxyl compound shown by the following formula (I): 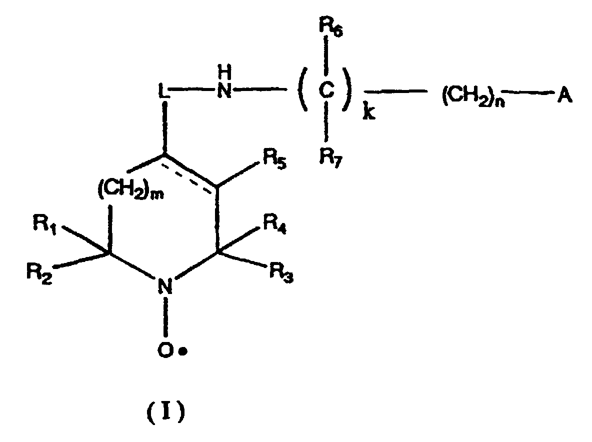 wherein L represents -CO- or -alkylene-CO-, R₁, R₂, R₃, and R₄ individually represent alkyl groups having 1-4 carbon atoms, R₅ represents a hydrogen atom, an alkyl group having 1-4 carbon atoms, carboxyl group, or lower alkoxycarbonyl group, m and k individually represent 0 or 1, n represents an integer from 1 to 4, R₆ represents a methyl group or ethyl group, R₇ represents a hydrogen atom, a methyl group, ethyl group, or carboxyl group, A represents an aromatic group having a radioactive isotopic element, and 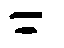 represents either a single bond or a double bond when m is 0, or a single bond when m is 1.

9. The labeled nitroxyl compound according to claim 8, wherein A represents one of the groups shown by the following formulas: 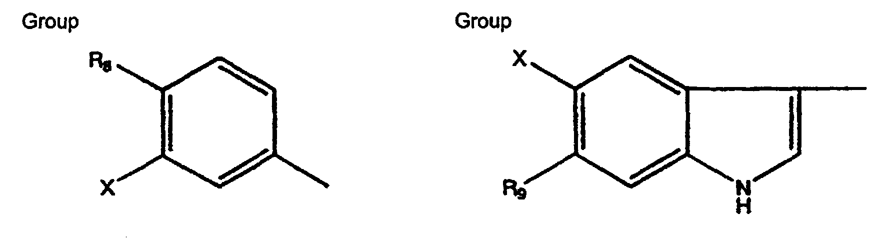 wherein R₈ and R₉ represent a hydrogen atom, a methyl group, ethyl group, hydroxyl group, or carboxyl group, and X represents a radioactive isotope of a halogen atom.

10. An intermediate used to prepare the labeled nitroxyl compound according to claim 9 which is shown by the following formula (II): 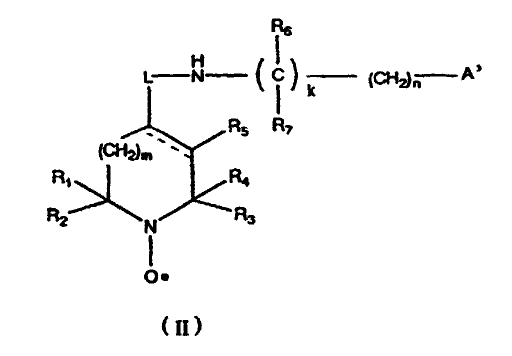 wherein L represents -CO- or -alkylene-CO-, R₁, R₂, R₃, and R₄ individually represent alkyl groups having 1-4 carbon atoms, R₅ represents a hydrogen atom, an alkyl group having 1-4 carbon atoms, carboxyl group, or lower alkoxycarbonyl group, m and k individually represent 0 or 1, n represents an integer from 1 to 4, R₆ represents a methyl group or ethyl group, R₇ represents a hydrogen atom, a methyl group, ethyl group, or carboxyl group, A' represents an aromatic group having a group which can be replaced by a radioactive isotopic element, and 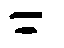 represents either a single bond or a double bond when m is 0, or a single bond when m is 1.

11. The intermediate according to claim 10, wherein A' represents one of the groups shown by the following formulas: 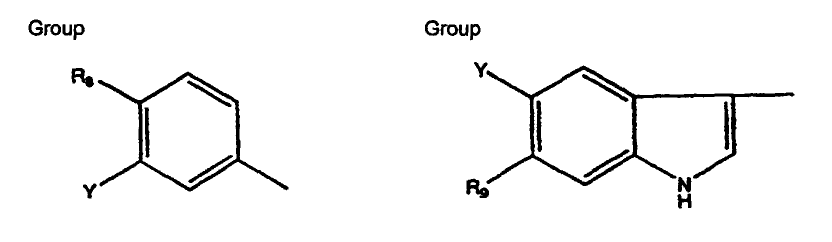 wherein R₈ and R₉ represent a hydrogen atom, a methyl group, ethyl group, hydroxyl group, or carboxyl group, and Y represents a stable isotope of a halogen atom.

12. The intermediate according to claim 10, wherein A' represents one of the groups shown by the following formulas: 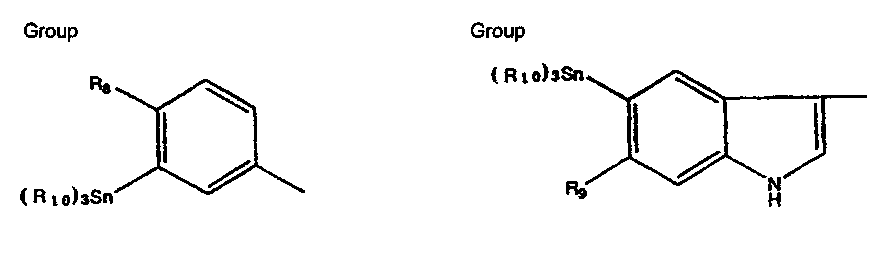 wherein R₈ and R₉ represent a hydrogen atom, a methyl group, ethyl group, hydroxyl group, or carboxyl group, and R₁₀ represents an alkyl group having 1-4 carbon atoms.
